# NEUE EUROPÄISCHE PATENTSCHRIFT

(11) **EP 2 358 642 B2**
(45) Veröffentlichungstag und Bekanntmachung des Hinweises auf die Entscheidung über den Einspruch: **07.12.2022**
(45) Hinweis auf die Patenterteilung: 19.06.2019
(21) Anmeldenummer: 09756716.8
(22) Anmeldetag: 16.11.2009
(51) Int. Cl.: C02F 1/42, C02F 101/16, C02F 1/28, A61K 33/00

(54) **KLINOPTILOLITH ODER KLINOPTILOLITHHALTIGE GEMISCHE**
CLINOPTILOLITE OR CLINOPTILOLITE COMPRISING MIXTURES
CLINOPTILOLITE OU MÉLANGES COMPRENANT DU CLINOPTILOLITE

(30) Priorität: 21.11.2008 DE 102008043967; 21.11.2008 DE 102008043959
(43) Veröffentlichungstag der Anmeldung: 24.08.2011
(73) Patentinhaber: Froximun AG, 38838 Schlanstedt (DE)
(72) Erfinder: STEIMECKE, Günter, 38855 Wernigerode (DE); HOFFMANN, Steffen, 38838 Schlanstedt (DE); GÖRNER, Thomas, 38838 Schlanstedt (DE)
(74) Vertreter: Sperling, Thomas
(86) Internationale Anmeldenummer: PCT/EP2009/065221
(87) Internationale Veröffentlichungsnummer: WO 2010/057849

(56) Entgegenhaltungen:
- EP-B1- 1 942 914
- WO-A2-2008/064249
- US-A1- 2005 031 708
- US-A1- 2005 106 267
- US-A1- 2008 044 548
- US-B1- 7 108 784
- SPRYNSKYY M ET AL: "Ammonium sorption from aqueous solutions by the natural zeolite Transcarpathian clinoptilolite studied under dynamic conditions" JOURNAL OF COLLOID AND INTERFACE SCIENCE, ACADEMIC PRESS, NEW YORK, NY, US, Bd. 284, Nr. 2, 15. April 2005 (2005-04-15), Seiten 408-415, XP004787391 ISSN: 0021-9797

## Beschreibung

Die Erfindung betrifft Klinoptilolith oder klinoptilolithhaltige Gemische, wobei das Klinoptilolith oder die klinoptilolithhaltigen Gemische durch Zerkleinerung der Teilchen mechanisch modifiziert und aktiviert sind, zur Verwendung als Medizinprodukt zur Aufnahme und zur Ausleitung von Aminen und/oder deren Salzen aus dem Verdauungstrakt oder dem Hautbereich von Mensch und Tier. Amine leiten sich strukturell von Ammoniak ab, wobei ein, zwei oder drei Wasserstoffatome des Ammoniaks durch gleiche oder verschiedene organische Reste substituiert sein können. Amine und deren Salze sind dem Chemiker seit langer Zeit bekannt und werden in vielfältiger Weise im Laboratorium sowie für technische Syntheseprozesse genutzt. Amine sind ebenfalls im menschlichen oder tierischen Organismus enthalten. Sie dienen in unterschiedlicher Weise als Steuer- und als Regulationssubstanzen für eine zum heutigen Zeitpunkt kaum noch überschaubare Zahl biologischer Vorgänge. Eine Einteilung in schädliche, den Körper eher belastende oder gar harmlose Substanzen kann aus diesem Grunde nicht getroffen werden und ist weder zweckmäßig noch sinnvoll. Man muss im Grunde jede einzelne Substanz sowie deren biologische Wirkung konkret bestimmen und in den für den Organismus relevanten Konzentrationen betrachten, um hinreichend verlässliche Aussagen über schädliche oder eher unschädliche Wirkungen einer bestimmten Konzentration treffen zu können. Des Weiteren gelangt eine permanent steigende Anzahl von Aminen, die als arzneilich wirksame Substanzen beispielweise in Form von blutdrucksenkenden Substanzen in pharmazeutischen Produkten verabreicht werden, oder in Form von Pflanzenschutzmitteln oder deren Rückständen spurenweise oder als Verunreinigung von Nahrungsmitteln in den menschlichen Körper. Eine industrielle, chemische Weiterverarbeitung von Aminen führt unter anderem zur Synthese von Azofarbstoffen: Man findet diese Klasse von Farbstoffen fast überall im menschlichen Lebensraum, angefangen von Beschriftungen technischer Anlagen über die Verpackung von Produkten bis hin zum Anfärben von Lebensmitteln, manchmal spurenweise, manchmal in größerer Menge. Andere Aminquellen stellen Koch-, Back-, Brat-, Grill- und Frittiervorgänge dar, die üblicherweise zur Zubereitung von Speisen genutzt werden. Hier handelt es sich vor allem um thermische Vorgänge. Die Amine werden gebildet durch Umsetzung von Aminosäuren mit Kohlenhydraten, Fetten und weiteren Stoffen in der Hitze. Aber auch Pflanzen sowie deren Inhaltsstoffe enthalten bedeutende Mengen an Aminen. Hier sei die große Gruppe der Alkaloide, der Sucht- und Rauschmittel sowie der Pilzgifte genannt.

Eine weitere bedeutende Quelle für Amine im menschlichen oder tierischen Körper stellt die Verdauung dar: Eiweiße werden unter den anaeroben Bedingungen des Darms unter dem Einfluss bakterieller Acylasen in kleinere Peptidsequenzen und schließlich zu Aminosäuren aufgespalten, die anschließend normalerweise via Darm resorbiert und im Organismus verwertet werden. Ein Teil dieser Aminosäuren wird aber unter dem Einfluss des von vielen Bakterien gebildeten Ferments *Aminosäuredecarboxylase* in Kohlendioxid und Amin gespalten oder über andere Stufen weiter abgebaut. In der Fachliteratur fasst man derartige Amine zweckmäßig unter der Sammelbezeichnung "biogene Amine" zusammen.

Eine weitere bedeutende Aminquelle ist die mikrobielle Aktivität: Alle Lebensmittel, die durch Einsatz von Mikroorganismen erzeugt beziehungsweise hergestellt worden sind, enthalten Amine. Hierzu zählen beispielsweise Käse, Wein, Sauerkraut, Bier aber auch Milchprodukte wie Jogurt und Kefir. Aber auch bei der Lagerung von Fleisch, Fisch, Wurst, Geflügel und Wild entstehen Amine. Der Anteil an Aminen steigt für gewöhnlich stark an, sofern Lebensmittel überlagert, ungenießbar oder verdorben sind. Hier geht neben der zunehmenden mikrobiologischen Belastung eine anfangs tolerierbare Aminkonzentration in eine den Körper belastende oder toxische Aminkonzentration über. Will man überflüssige Mengen belastender, bakterieller Amine vermeiden, sollte man Lebensmittel stets frisch erzeugt zubereiten und auch verzehren. Kritisch wird die Situation jedoch, sofern die betreffenden bakteriellen Amine im menschlichen oder tierischen Organismus eine bestimmte, spezifische regelnde Körperfunktion, zum Beispiel die eines Gewebshormons, ausüben. In diesen Fällen ist der Organismus gezwungen, das betreffende, im Speisebrei enthaltene Amin chemisch vollständig zu verändern, damit es nicht durch Resorption über die Darmschleimhaut in den Körper aufgenommen wird. Es käme sonst zu unkontrollierbaren Reaktionen, der Organismus verlöre in den betroffenen Geweben die biochemische Handlungsinitiative. Hierzu dient das Fermentsystem *Monoaminoxidase* beziehungsweise *Diaminoxidase.* Die hinreichend hohe Aktivität dieses Fermentsystems in der Darmschleimhaut, das eine große Zahl von hauptsächlich primären Aminen mittels Wasserstoffperoxid durch oxidative Deaminierung zur betreffenden Carbonylverbindung und Ammoniak abzubauen in der Lage ist, ermöglicht höheren Organismen, vorzugsweise sich vor biogenen Aminen zu schützen. Diese Reaktion ist eine oxidative Deaminierung unter Ammoniak-Abspaltung. Sie ist die Umkehrung der reduktiven Aminierung, die unter der Bezeichnung Leukart-Wallach-Reaktion in der Chemie seit etwa 100 Jahren bekannt ist. Durch Alterungsvorgänge, Enzymdefekte, durch krankhafte Veränderungen der Darmschleimhaut, durch eine Vielzahl sich im Verkehr befindender aminooxidasehemmender Medikamente sowie bestimmter bei der Lebensmittelherstellung zugelassener Hilfs- und Konservierungsstoffe kann es bei Mensch und Tier zur Reduktion der Diamino- beziehungsweise Aminooxidaseaktivität, in seltenen Fällen sogar zum nahezu vollständigen Verlust der Diamino- beziehungsweise Aminooxidaseaktivität in Teilbereichen des Darms kommen. Die Betroffenen erkranken zum Beispiel im Falle der Aufnahme unkontrollierter Mengen von 2-(4'-Imidazolyl)-ethylamin, welches im menschlichen Körper als Gewebshormon sowie als Neurotransmitter wirkt, an schweren Darmentzündungen oder an multipler Lebensmittelunverträglichkeit. Der Krankheitsverlauf erstreckt sich zum Teil über Jahre, manchmal Jahrzehnte, und ist durch die besondere Schwere der Erkrankung für die Betroffenen auch mit einer starken psychischen Belastung verbunden. Die Anzahl der Erkrankten hat sich in den letzten dreißig Jahren stark erhöht. Die häufiger vorkommende leichtere Form der Erkrankung bleibt zumeist in seiner Ursache unerkannt und führt in Abständen immer wieder zu zum Teil schmerzhaften und recht unangenehmen Verdauungsproblemen. Im menschlichen und im tierischen Körper spielt 2-(4'-Imidazolyl)-ethylamin eine zentrale Rolle bei allergischen Reaktionen und ist an der Abwehr körperfremder Stoffe und von Mikroorganismen über das Immunsystem beteiligt. Sowohl im Gastrointestinaltrakt bei der Regulation der Magensäureproduktion als auch im Zentralnervensystem bei der Regulation des Schlaf-Wach-Rhythmus ist 2-(4'-Imidazolyl)-ethylamin eine sehr wichtige Substanz. Das 2-(4'-Imidazolyl)-ethylamin wird in Mastzellen, basophilen Granulozyten und in Nerverzellen gespeichert. Aus den Mastzellen und den basophilen Granulozyten kann 2-(4'-Imidazolyl)-ethylamin im Bedarfsfall sehr schnell freigesetzt werden. Dies ist zum Beispiel bei allergischen Reaktionen oder mechanischen Verletzungen der Fall. Die physiologische Wirkung von 2-(4'-Imidazolyl)-ethylamin besteht in einer Gefäßerweiterung der kleinen Gefäße, die sich durch Hautrötungen, Erhöhung der Gefäßpermeabilität und Kontraktion der glatten Muskulatur in Bronchien, Darm, Uterus und den großen Gefäßen äußert. Die unerwünschten Wirkungen bei allergischen Reaktionen bestehen in Kopfschmerzen, laufender Nase, Atemwegsbeschwerden bis hin zu Asthma, Hautreaktionen wie Juckreiz und Quaddel-Bildung. Auch wird die Herzfunktion durch 2-(4'-Imidazolyl)-ethylamin beeinflusst. Es kann Tachykardie auftreten oder es kann zu Extrasystolen kommen. Der Blutdruck fällt deutlich. Die Auswirkungen auf den Magen-Darmtrakt besteht im Auftreten weicher Stühle und von Durchfall.

Die wichtigste Funktion des körpereigenen 2-(4'-Imidazolyl)-ethylamins besteht in seiner Beteiligung an der Abwehr körperfremder Stoffe und seiner pathologischen Beteiligung an der Symptomatik von Allergien und Asthma. Ebenfalls ist 2-(4'-Imidazolyl)-ethylamin eine Mediatorsubstanz bei Entzündungen und Verbrennungen. Hierbei führt 2-(4'-Imidazolyl)-ethylamin zu Juckreiz und Schmerz, Kontraktion der glatten Muskulatur in den Bronchien und den großen Blutgefäßen. Eine gesteigerte Permeabilität der Gefäßwände führt zur Nesselsucht. 2-(4'-Imidazolyl)-ethylamin führt weiterhin zur Freisetzung von Adrenalin. Eine Resorption von 2-(4'-Imidazolyl)-ethylamin aus dem Darm versucht der Körper durch eine hohe Aktivität des Enzyms Monobeziehungsweise *Diaminoxidase* und durch einen möglichst vollständigen Abbau von 2-(4'-Imidazolyl)-ethylamin zu verhindern.

Zwei weitere Amine, die durch mikrobielle Aktivität im Darm während der Verdauungsvorgänge entstehen können, sind 2-Phenyl-ethylamin und 2-(4'-Hydroxyphenyl)-ethylamin. Kommt es zur Resorption von 2-Phenyl-ethylamin, so steigt der Blutzuckerspiegel an. 2-Phenyl-ethylamin ist gleichzeitig auch ein Amphetaminderivat und somit ein Amin, das süchtig macht, zu gesteigertem Appetit führt und mit der Zeit durch die andauernde Steigerung des Blutzuckerspiegels den Typ II-Diabetes befördern kann. Üblicherweise kommt es in größerer Menge in Schokolade vor, der menschliche Körper kann es unter bestimmten Bedingungen selbst produzieren. Es entsteht im Darm durch bakteriellem Abbau der Aminosäure Phenylalanin. Das 2-(4'-Hydroxyphenyl)-ethylamin steigert durch Anregung des Sympatikus den Blutdruck sehr stark. Systolische Blutdruckwerte von 250 bis 300 Millimeter Quecksilbersäule sind durch die Wirkung von 2-(4'-Hydroxyphenyl)-ethylamin keine Seltenheit. Die Gefahren für die Gesundheit sind leicht vorstellbar und werden meist unter der Bezeichnung hypertone Krise zusammengefasst. 2-(4'-Hydroxyphenyl)-ethylamin entsteht aus der Aminosäure Thyrosin wiederum durch den bakteriellen Abbau unter dem Einfluss von Decarboxylasen.

Ein weiteres, sehr bedeutendes, biologisch wirksames Amin ist das Dimethylamin, ein sekundäres Amin. Es entsteht in verhältnismäßig großer Menge im Muskelgewebe und gelangt durch den Verzehr hauptsächlich von Fisch unmittelbar in den Darm und/oder entsteht teilweise durch bakteriellen Abbau während der Verdauung häufig über die Zwischenstufe Sarkosin auch infolge von Transmethylierungsreaktionen, zum Beispiel des Methionins. Gesteigerte Konzentrationen an Dimethylamin beeinträchtigen neurophysiologische Funktionen. Besonders bei Personen mit eingeschränkter Nierenfunktion und im höheren Lebensalter ist dieser Sachverhalt bedeutsam. Neben seiner blutdrucksenkenden Eigenschaft besitzt Dimethylamin die Fähigkeit, mit Nitrit, welches durch mikrobielle Reduktion aus Nitrat des Trinkwassers oder verzehrtem Gemüse im Darm entsteht, zu reagieren. Bei dieser Reaktion, die bei pH-Werten um pH 5,0 durch chemische Reaktion spontan und durch eine große Zahl von Bakterien-Spezies selbst bei einem annähernd neutralen pH um pH 7,2 katalysiert wird, entsteht Dimethylnitrosamin, eine Substanz, die als stark krebserregende Substanz zur Gruppe der Nitrosamine zählt. Der überwiegende Teil dieser Nitrosamine hat die Eigenschaft, im Tierexperiment Tumoren zu erzeugen. Somit geriet Dimethylamin bereits vor 30 bis 40 Jahren in Verdacht, als Vorstufe für das gefährliche Dimethylnitrosamin als ein potentielles Präkanzerogen im menschlichen und tierischen Körper zu wirken. Der Abbau von Dimethylamin mittels Aminooxidasen der Darmschleimhaut ist nicht möglich, so dass die gesamte Menge an Dimethylamin, die sich im Darm befindet oder dort entsteht, resorbiert und über die Blutbahn im ganzen Körper verteilt wird. Durch die Aktivität der Nieren wird Dimethylamin zum Bestandteil des Urins gesunder Menschen und kann auf diese Weise aus dem Körper ausgeschieden werden. Es lässt sich am günstigsten mittels Gaschromatographie, ¹H-NMR-Spektroskopie oder photometrisch im Urin nachweisen und bestimmen. Durch den Abbau von methyliertem Arginin entsteht die Hauptmenge Dimethylamin. Der gesunde Mensch scheidet pro Tag über den Urin durchschnittlich etwa 17,5 Milligramm Dimethylamin aus, Männer mit 21,2 Milligramm mehr als Frauen, die durchschnittlich 13,5 Milligramm ausscheiden.

Ein weiteres, bedeutendes sekundäres Amin ist das Pyrrolidin. Es kann durch bakterielle Decarboxylierung der Aminosäure Prolin entstehen. Prolin ist als Aminosäure Bestandteil des Eiweißes. In Analogie zu Dimethylamin entsteht aus Pyrrolidin mit Nitrit Nitrosopyrrolidin, eine ebenfalls stark krebserregende Verbindung. In einigen Organismen wurde das Pyrrolidin gefunden.

Eine Reihe weiterer Krebs auslösender Amine entsteht im Darm unter den stark reduktiven Bedingungen während der Verdauung. Vor allem aus der Gruppe der Azofarbstoffe bilden sich durch Spaltung der Azogruppe aromatische Amine, wie Benzidin, Anilin und Naphthylamin, in Substanz oder in Form substituierter Abkömmlinge. Für eine Reihe dieser aromatischen Amine besteht inzwischen ein generelles Herstellungs- und Verwendungsverbot. Die aus diesen Stoffen chemisch zugänglichen Azofarbstoffe dürfen bis zum heutigen Tage international hergestellt und auch gehandelt werden. Auf die Herstellung eines Teils dieser potentiell gefährlichen Azofarbstoffe hat die chemische Industrie in Deutschland seit einigen Jahren freiwillig verzichtet. Über Importe von Fertigerzeugnissen können jedoch alle in Frage kommenden Azofarbstoffe auf den Binnenmarkt gelangen, sofern in den Herstellungs- und Erzeugerländern nicht ähnliche, nach Möglichkeit freiwillige Herstellungsbeschränkungen bestehen.

In Analogie zum Darm kann es natürlich auch über die Haut zur Aufnahme von Stoffen, insbesondere von Aminen, in den Körper kommen:
Geringe Verletzungen, Insektenstiche, Schürfwunden, aber auch stärkere Verletzungen können durch infektiöse Keime besiedelt sein. Die Wunden entzünden sich und manche Mikroorganismen sind in der Lage, auch in dieser Umgebung Amine, insbesondere das 2-(4'-Imidazolyl)-ethylamin, freizusetzen. Nesselsucht ist häufig die unmittelbare Folge. Die Aktivität der aminabbauenden Oxidasen ist im Bereich der Haut vergleichsweise geringer als in der Darmschleimhaut. Teilweise sind die Mikroorganismen in der Lage, zusätzlich noch Giftstoffe zu produzieren, die in das umgebende, gesunde Gewebe eindringen können und auf diese Weise das Gewebe zerstören und zur Lyse führen. Das lysierte Gewebe seinerseits dient den Mikroorganismen für deren weitere Entwicklung und Vermehrung als Grundlage. Die Wunde vergrößert sich weiter. Die Infektion nimmt zu. In vielen Fällen werden derartige Infektionen durch *Staphylococcus aureus,* einem Bakterium, das auf der Haut oder in der Nase des Menschen üblicherweise anzutreffen ist, hervorgerufen. Viele Spezies von *Staphylococcus aureus* sind gegenüber Antibiotika inzwischen resistent. Die Resistenz hat zur Folge, dass eine Behandlung mit einem Antibiotikum oder mehreren Antibiotika in Kombination wenig oder nicht mehr anschlägt und die Bakterien in der Wunde sich ungehindert weiter vermehren können. Die Wunden entwickeln sich auf diese Weise immer weiter und dringen immer tiefer ins Gewebe ein, bis schließlich klinisch ein wirksames Antibiotikum gefunden ist. Existiert kein wirksames Antibiotikum oder wird ein solches nicht rechtzeitig gefunden, versterben die Betroffenen zumeist an eintretender Sepsis.

Das disulfidische Cystamin, ebenfalls ein biogenes Amin, hat die Eigenschaft, die Blutgerinnung zu hemmen und in höherer Konzentration die Blutgerinnung vollständig außer Kraft zu setzen. In den 1960er Jahren gab es Bestrebungen, diese Eigenschaft für die Entwicklung von neuen Thrombozytenaggregationshemmern zu nutzen, jedoch scheiterten diese Versuche aufgrund der Hautabzessbildung und wegen sich ergebender Lungenprobleme. Das Cystamin führt darüber hinaus zu Appetitlosigkeit, Schläfrigkeit, erhöhtem Speichelfluss und senkt den Glucosespiegel des Blutes.

Eine Reihe von Erkrankungen ist verbunden mit spontanen Blutungen, zum Beispiel des Darms oder anderer Schleimhäute. Häufig auftretende spontane Blutungen von Schleimhäuten, zum Beispiel in Darm, Nase und Lunge, können einen infektiösen Ursprung haben. Kommt es nach Infektionen zur Freisetzung von Cystamin in hoher Konzentration und/oder hat der Mensch krankheitsbedingte Monoamin- beziehungsweise Diaminoxidaseschwächen bezüglich Cystamin in den betroffenen Geweben, können spontane Blutungen oder Einblutungen ins Gewebe durch lokale Außerkraftsetzung der Blutgerinnung auftreten. Sehr starke Blutungen der Schleimhäute treten bei den in den Tropen sporadisch immer wieder aufflackernden hämorrhagischen Fiebern auf. Lassafieber, Ebolafieber und Denguefieber seien ausdrücklich genannt. Aber auch diesbezügliche nosokominale Infektionen spielen eine immer weiter zunehmende, nicht zu unterschätzende Rolle und erfordern bei der Behandlung allerhöchste hygienische Sicherheitsmaßnahmen.

Tryptamin hat die Eigenschaft, wenn es in die Blutbahn über die oben bereits beschriebenen Wege gelangen kann, bei den Betroffenen Alpträume auszulösen. Dies ist nicht verwunderlich, denn Tryptamin ist dem LSD = Lysergsäurediethylamid strukturell nahestehend. Trypramin ist nicht selten natürlicher Bestandteil schwerer Rotweine und auch manchmal in guten Lagerweinen zu finden.

Wie aus den bisherigen Ausführungen hervorgeht, haben Amine die Fähigkeit, den Stoffwechsel und damit die Gesundheit von Mensch und Tier zu beeinflussen. Von wesentlicher Bedeutung ist in diesem Zusammenhang die Erkenntnis, dass bei Reduktion der Enzymaktivität der Monoamin- und Diaminoxidase oder bei der Entstehung großer Mengen von Aminen es im Darm oder über die Haut zu einer unkontrollierten Aufnahme einer ganzen Reihe von Aminen in den menschlichen oder tierischen Körper kommen kann. Dann kann es zu den oben beschriebenen Folgen für die Gesundheit kommen. Eine Reihe von Aminen wird im Darm nicht oder kaum abgebaut, andere werden im Darm bei der Verdauung sogar erst erzeugt. Hieraus ergibt sich die Aufgabenstellung, ein Verfahren zu entwickeln, mit dem es gelingt, die Aufnahme von Aminen über den Darm und über die Haut zu reduzieren.

Aus dem Stand der Technik sind Verfahren zur Entfernung von anorganischen Ammoniumsalzen bekannt. Die US 7,108,784 B1 beschreibt ein System für die Entfernung von Ammoniumionen (NH₄⁺) aus einem wässigen Medium, das unter anderem eine Ammoniumabsorptionseinheit mit einem Anteil an natürlichem Zeolith, der das wässrige Medium kontaktiert, sowie eine Ammoniumelutionseinheit für das Eluieren des Ammoniums aus dem Zeolith mit einer gesättigten Kochsalzlösung umfasst. Als bevorzugtes natürliches Zeolith wird Klinoptilolith verwendet.

Aus der Druckschrift SPRYNSKYY M ET AL: "Ammonium sorption from aqueous solutions by the natural zeolite Transcarpathian clinoptilolithe studied under dynamic conditions", JOURNAL OF COLLOID AND INTERFACE SCIENCE, ACADEMIC PRESS, NEW YORK, NY, US, Bd. 284, Nr. 2, 15. April 2005 (2005-04-15), Seiten 408-415, XP004787391, ISSN: 0021-9797, ist eine Studie der Ammoniumionen-Aufnahme aus synthetischen wässrigen Lösungen bekannt, die mittels roher und vorbehandelter Formen von natürlichem Klinoptilolith unter dynamischen Bedingungen stattfindet. Die Ammonium-Aufnahme am Zeolith erfolgt durch den Austausch mit Na⁺-, Ca²⁺- und K⁺-Ionen. Obwohl bei Na⁺-Ionen ein leichterer Austausch sowohl für Wasserstoffals auch für Ammonium-Ionen beobachtet werden kann, steigt die Bedeutung von Ca²⁺-Ionen mit der Zeolith-Sättigung durch NH₄⁺-Ionen. Die maximale Sorptionskapazität des Klinoptiloliths gegenüber NH₄⁺-Ionen, die unter dynamischen Bedingungen erhalten wird, ist wesentlich höher als die Sorptionskapazität, die unter statischen Bedingungen gemessen wurde. Die Ammoniumentfernung verbesserte sich mit der Nutzung von feineren Klinoptilolith-Fraktionen bis zu 0,35 mm. Wasserstoff-Ionen verdrängten austauschbare Kationen des Klinoptiloliths in destilliertem Wasser (NatriumIonen) und in Salzsäure (Natrium-, Kalium- und Calcium-Ionen) und zerstörten im letzteren Fall die Zeolithgerüststruktur. Die Vorbehandlung des Klinoptiloliths zeigte die besondere Bedeutung des Ionenaustauschmechanismus. So konnte der Vorteil einer vorherigen Behandlung des Klinoptiloliths mit Natriumchlorid gezeigt werden, wobei sich die Ammoniumentfernung gegenüber anderen Techniken verbesserte.

Die US 2008/044548 A1 beschreibt eine Tierfutterzusammensetzung, die im Wesentlichen aus einem Kationenaustauscher und aus einem acidogenen Material besteht, wobei der Kationenaustauscher in einer Menge von bis etwa 2,5 Gewichtsprozent beigemischt wird. Ein mit dieser Zusammensetzung gefüttertes Tier scheidet ein Produkt aus, welches Ammoniumionen aufweist, die am Kationenaustauscher gebunden sind. Zu den geeigneten Kationenaustauschern zählt auch Klinoptilolith, das einen erheblichen Anteil an austauschbarem Kalzium und Magnesium enthält.

Die US 2005/106267 A1 beschreibt die medizinische Anwendung von natürlichen Zeolithen für die Behandlung, Vorbeugung und Linderung von Schädigungen durch Konzentrationen von Ammoniak, Mecaptanen, Schwermetallen und anderen Giftstoffen infolge oraler Aufnahme bei Menschen und Tieren.

Die US 2005/0031708 A1 beschreibt einen Zeolith als ein Pulver in einem Gemisch mit verschiedenen Vitaminen, wobei das Gemisch zur Entgiftung des menschlichen Körpers vorgesehen ist und der Zeolith eine durchschnittliche Partikelgröße von 1 bis 2000 Mikrometer aufweist, bevorzugt über 500 Mikrometer. Das Zeolithmaterial dient der Reduzierung von aufgenommenen, bakteriell oder metabolisch entstehenden Giftstoffen. Insbesondere findet das Gemisch Anwendung im Zusammenhang mit einer Alkoholvergiftung.

Die WO 2008/064249 A beschreibt ein Verfahren und eine Zusammensetzung vorrangig für die Entfernung von Ammoniumsalzen aus Trinkwasser unter Nutzung eines natürlichen Zeoliths. Dafür wird das natürliche Zeolithmaterial zunächst mit einer ausreichenden Menge von einem Übergangsmetall beladen, um den Zeolith zu stabilisieren. Unter bestimmten, nicht näher erläuterten Bedingungen können laut der WO 2008/064249 A mit der beschriebenen Methode auch sekundäre und tertiäre Amine entfernt werden.

Die Aufgabe der Erfindung besteht insbesondere darin, eine Möglichkeit zur Herstellung eines pharmazeutischen Mittels für die wirksame Reduktion der Konzentrationen von 2-(4'-Imidazolyl)-ethylamin im Darm und auf der Haut bereitzustellen.

Die Lösung der der Erfindung zugrunde liegenden Aufgabe besteht in Klinoptilolith oder klinoptilolithhaltigen Gemischen, wobei das Klinoptilolith oder die klinoptilolithhaltigen Gemische durch Zerkleinerung der Teilchen mechanisch modifiziert und aktiviert sind, zur Verwendung als Medizinprodukt zur Aufnahme und Ausleitung von 2-(4'-Imidazolyl)-ethylamin und/oder dessen Salzen, das/die in wässriger Lösung oder wässriger Lösung oder wässriger Suspension im Verdauungstrakt oder dem Hautbereich von Mensch und Tier vorkommt/vorkommen.

Grundlage für diese Verwendung, jedoch nicht Gegenstand der Erfindung, ist ein Verfahren zur Reduktion der Konzentration von Aminen und deren Salzen, wobei die Amine die allgemeine chemische Formel I **R¹R²R³N** und deren Salze die Formel II **R¹R²R³N-H** aufweisen, bei dem die Amine und deren Salze in wässriger Lösung oder wässriger Suspension vorliegen und mit Klinoptilolith oder klinoptilolithhaltigen Gemischen in Kontakt gebracht werden. Erfindungsgemäß wird der natürlich vorkommende Zeolith Klinoptilolith verwendet. Klinoptilolithe sind hydratisierte Alumosilicate (Zeolithe) mit einer Bruttoformel (Ca, K₂, Na₂, Mg)₄Al₈Si₄₀O₉₆ ^{∗} 24 H₂O.

Die natürlichen Vorkommen bestehen zu 84 Prozent aus reinem Klinoptilolith. Die weitere mineralogische Zusammensetzung wird ergänzt durch:

| | |
|---|---|
| • Cristobalit: | 8 Prozent; |
| • Feldspat: | 3 bis 4 Prozent; |
| • Illit: | 4 Prozent; |
| • Quarz: | Spuren; und |
| • Carbonatminerale. | |

Chemisch ist das Mineral aus den folgenden Oxiden zusammengesetzt:

| | |
|---|---|
| • SiO₂ | 65 bis 71,3 Prozent; |
| • Al₂O₃ | 11,5 bis 13,1 Prozent; |
| • CaO | 2,7 bis 5,2 Prozent; |
| • K₂O | 2,2 bis 3,4 Prozent; |
| • Fe₂O₃ | 0,7 bis 1,9 Prozent; |
| • MgO | 0,6 bis 1,2 Prozent; |
| • Na₂O | 0,2 bis 1,3 Prozent und |
| • TiO₂ | 0,1 bis 0,3 Prozent. |

Folgende physikalische Daten wurden ermittelt:

| | |
|---|---|
| Erweichungstemperatur: | 1260 °C; |
| Schmelztemperatur: | 1340 °C; |
| Dichte: | 2,2 bis 2,5 g/cm³; |
| Druckfestigkeit: | 33 Mpa; |
| Porösität: | 32 bis 40 Prozent; |
| Porendurchmesser: | 0,4 nm und |
| Mohssche Härte: | 2,5 bis 3,5. |

Der Klinoptilolith ist grau-grün gefärbt und geruchlos. Das Grundskelett des Klinoptilolith-Kristallgitters besteht aus SiO₂-Tetraedern und Al₂O₃. Das Verhältnis von Silizium zu Aluminium liegt beim Klinoptilolith bei 4:1. Die über Sauerstoffbrücken gebildeten Kristallstrukturen lassen Poren und Kanäle entstehen. Die Oberfläche des Klinoptiloliths ist ganz außergewöhnlich groß, da im Inneren des Kristallgitters in allen drei Raumrichtungen verbundene Hohlräume existieren. Durch den Austausch von vierwertigen Siliziumatomen durch dreiwertige Aluminiumatome entsteht eine Kationenaustauschkapazität. Dieser Austausch bewirkt einen negativen Ladungsüberschuss. Die negativen Ladungen der Silizium/Aluminium-Tetraeder werden im Inneren durch bewegliche, in den Hohlräumen eingelagerte positive Ionen der Alkali- und Erdalkalielemente (Ca, K, Na, Mg) sowie durch größere Mengen eingelagerten Wassers kompensiert. Ein charakteristisches Merkmal der Zeolithe ist die selektive Austauschfähigkeit der gebundenen Ionen gegen andere (Selektivität: Cs⁺>Pb²⁺>NH₄⁺>Cu²⁺>Hg²⁺>Cd²⁺>Ni²⁺>Co2⁺, NH₄⁺>K⁺>Mg²⁺>Ca²⁺). Seine lonenaustauscherkapazität beträgt 1,2 bis 1,5 mol pro kg. Ca²⁺ und Mg²⁺ sind dabei die dominantesten austauschbaren Kationen des Zeoliths. Sie können durch andere Elemente ausgetauscht werden, wie zum Beispiel durch schädliche Elemente, wie Blei, Quecksilber und andere Schwermetalle, sowie durch das Ammonium.

Auch kleineren und mittleren Molekülen gelingt es, in diese Hohlräume über die Kanäle einzudringen und die vorhandenen, beweglichen Ionen aus ihren Positionen zu verdrängen. Zu diesen Molekülen zählen überraschenderweise auch die zuvor erwähnten Amine der Formel I und Salze protonierter Amine der Formel II. Besonders geeignet ist das oben genannte Verfahren für die Reduktion der Konzentration von Aminen und deren Salzen, bei denen die drei Reste R¹, R² und R³ der Formeln I und II maximal zwei Wasserstoffatome und maximal drei gleiche oder verschiedene Alkyl-, Alkenyl-, Aryl-, Aralkyl-, Polyaryl- oder heterocyclische Reste sind, die ihrerseits durch gleiche oder verschiedene Reste, wie Alkyl-, Halogen-, NH₂-, Acyl-NH-, Aryl-NH-, Heterocyclyl-, -OH, -SH und dessen Oxidationsprodukte, -S-CH₃, -CO-NH₂, -O-CH₃, substituiert sein können. Vorzugsweise erfolgt die Anwendung des Verfahrens auf solche Lösungen oder Suspensionen, bei denen sich die Verbindungen der allgemeinen Formel I und der Formel II in der wässrigen Lösung oder der Suspension in Wasser durch Umsetzung mit Säure oder durch Deprotonierung in Wasser im chemischen Gleichgewicht miteinander befinden:

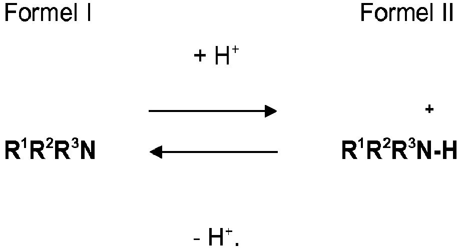

Zum besseren Verständnis des Vorganges sollte hinzugefügt werden, dass alle Amine in wässriger Lösung durch Umsetzung mit Wasser sich in Salze umwandeln und je nach pH-Wert mehr in der Aminform oder mehr in der Salzform vorliegen. Es handelt sich um eine Gleichgewichtsreaktion. Bei pH 6 bis pH 7 liegen die meisten leicht wasserlöslichen Amine vorzugsweise bereits zum größten Teil in der Salzform vor. Wenn in der vorliegenden Erfindung von Amin gesprochen wird, ist im Folgenden selbstverständlich immer auch das entsprechend korrespondierende Salz des Amins gemeint, welches in das Kristallgitter des Klinoptiloliths hauptsächlich eingebaut werden kann.

Erfindungsgemäß werden Klinoptilolith oder die klinoptilolithhaltigen Gemische vor dem Zusammenbringen mit den Aminen und/oder deren Salzen durch Zerkleinerung der Teilchen mechanisch modifiziert und aktiviert. In einer bevorzugten Ausführungsform der Erfindung weisen die Partikel des Klinoptilolith nach der Zerkleinerung eine Partikelgröße von 2 bis 16 µm, Zetapotenziale von 20 bis 26 mV und eine spezifische Oberfläche von 30 bis 60 m²/g auf.

Ein bevorzugtes Anwendungsgebiet des Verfahrens, welches nicht Teil der Erfindung ist, umfasst die Zubereitung von Speisen und die Herstellung von Lebensmitteln, um die Konzentration an Aminen mit der allgemeinen chemischen Formel I und/oder deren Salzen der Formel II in den Speisen oder Lebensmitteln zu reduzieren. Die Reduktion der Konzentration an Aminen mit der allgemeinen chemischen Formel I und/oder deren Salzen der Formel II kann geschehen, indem schon vor der Speiseneinnahme, zum Beispiel bei der Herstellung oder dem Verzehr der Lebensmittel, Klinoptilolith oder ein Klinoptilolithgemisch zugesetzt wird.

Möglich ist jedoch auch die separate Einnahme von Klinoptilolith in definierter Menge zu einem bestimmten Zeitpunkt, gegebenenfalls im Gemisch mit anderen Stoffen. Daher ergibt sich die Möglichkeit der Verwendung von Klinoptilolith oder klinoptilolithhaltigen Gemischen zur Herstellung von Medizinprodukten zur Aufnahme und Ausleitung von Aminen mit der allgemeinen chemischen Formel I **R¹R²R³N** und/oder deren Salzen der Formel II **R¹R²R³N-H**, die in wässriger Lösung oder wässriger Suspension im Verdauungstrakt oder dem Hautbereich von Mensch und Tier vorkommen. Gemäß der Konzeption der Erfindung beruht die Wirkung eines solchen Medizinproduktes einerseits darauf, dass durch dessen Einnahme dem Speisebrei von Mensch und Tier Klinoptilolith zugesetzt beziehungsweise dieser damit in Kontakt gebracht wird. Unter Speisebrei wird jenes Gemisch verstanden, das im Magen unter der Einwirkung von Verdauungssekreten, zum Beispiel Speichel und Magensaft, aus den aufgenommenen Speisen entsteht. Da der Speisebrei stets wasserhaltig ist, kommt es zu einer Bindung der Amine an das Klinoptilolith. Schwach gebundene Ionen, wie das Calcium oder das Magnesium, werden dabei an den Speisebrei der Verdauung abgegeben. Dieser Vorgang kann auch als lonenaustausch bezeichnet werden. Beim Klinoptilolith handelt es sich um ein Calciumaluminiumsilicat.

Andererseits wird das Klinoptilolith nach Einnahme über den Darm oder die Haut nicht resorbiert. Entsprechende Blutspiegel von Klinoptilolith sind nicht nachzuweisen. Die Wirkung von Klinoptilolith besteht somit insgesamt in der Bindung von Aminen beziehungsweise deren Salzen des Darms, die bei der Verdauung entstehen oder bereits im Speisebrei enthalten waren, an Klinoptilolith und die anschließende Ausscheidung über den Stuhl. Das Fehlen einer Resorption wurde durch Versuche mit Technetium-99 nachgewiesen. Es konnte keine Strahlung in den Organen nachgewiesen werden, die durch eine Resorption von Klinoptilolith-Partikeln, mit denen Technetium-99 fest gebunden war, hervorgerufen worden wäre. Durch die erfindungsgemäße Anwendung von Klinoptilolith wird der Organismus von Aminen, die hauptsächlich während der Verdauung oder der Wechselwirkung von Mikroorganismen mit körperlichem Gewebe entstehen, entlastet. Eine Reihe von Aminen haben ein relativ hohes Bindungsvermögen für Klinoptilolith. Dieses ist zum Teil deutlich gesteigert gegenüber Ammonium oder Schwermetallionen. Wie bereits erwähnt, kann das Klinoptilolith durch entsprechende mechanische Verfahren zuvor aktiviert und modifiziert sein, wobei die Partikel des Klinoptilolith vorzugsweise in der Weise zerkleinert werden, dass sie eine Partikelgröße von 2 bis 16 µm, Zetapotenziale von 20 bis 26 mV und eine spezifische Oberfläche von 30 bis 60 m²/g aufweisen. Bei Anwendung auf der Haut trocknet das Klinoptilolith und fällt schließlich gegebenenfalls gemeinsam mit dem Schorf ab. Zusammenfassend lässt sich feststellen, dass bei entsprechender Anwendung Klinoptilolith den Körper im Darm und/oder auf der Haut von Aminen entlastet.

Im Falle von Dimethylamin, das Bestandteil des Urins gesunder Menschen ist und welches durch bakteriellen Abbau aus Kreatin entstehen kann, kommt es durch die Anwendung von Klinoptlolith zu einer Entlastung von einem Stoff im menschlichen und tierischen Körper, der mit Nitrit unter physiologischen Bedingungen und sogar ohne Einflussnahme von Enzymen zu Dimethylnitrosamin reagiert. Diese Reaktion vollzieht sich auch *in vitro* bei pH-Werten um pH 5 spontan und wird bei pH-Werten um pH 7 durch sehr viele Bakterien auch enzymatisch katalysiert. Dimethylamin spielt aus diesem Grunde die Rolle eines Präkanzerogens. Die Nitritbildung vollzieht sich gleichfalls durch Beteiligung von Bakterien. Bakterien des Darms reduzieren Nitrat zu Nitrit. Nitrat entsteht aber auch durch die Verstoffwechselung von Stickstoffmonoxid, das der Körper zur Einstellung des Blutdrucks selbst herstellt. Es besteht zwar die Möglichkeit, die Nitrataufnahme durch nitratfreies oder nitratarmes Trinkwasser zu reduzieren, die Stickstoffmonoxidbildung lässt sich bisher jedoch nicht beeinflussen, so dass die mögliche Einflussnahme auf die Präkanzerogenese von der Seite Nitrat/Nitrit begrenzt erscheint.

Die Bindung von Dimethylammonium an Klinoptilolith ist außergewöhnlich hoch, überraschenderweise deutlich stärker als etwa die Bindung von Ammonium an Klinoptilolith. Diese neue Erkenntnis eröffnet die Möglichkeit, in den Prozess der Präkanzerogenese, also der Krebsentstehung, durch Verwendung von Klinoptilolith aktiv einzugreifen. Dimethylamin ist nach bisherigen Erkenntnissen gleichzeitig mengenmäßig das Hauptpräkanzerogen im menschlichen Körper. Die vorliegende Idee kann zur Entwicklung von Medizinprodukten genutzt werden, mit dem Ziel, den Körper mit Dimethylamin/Dimethylammonium zu entlasten, was allerdings nicht Gegenstand der Erfindung ist, und die Entstehung von Krebs durch die Beeinflussung der Präkanzerogenese zu verhindern. Andere potentielle Päkanzerogene, die auch mit Nitrit Nitrosamine bilden, werden durch Klinoptilolith überraschenderweise ebenfalls gebunden. Hierzu zählen Pyrrolidin, β-Hydroxypyrrolidin und Piperidin beziehungsweise deren Salze, was allerdings ebenfalls nicht Gegenstand der Erfindung ist. Die beiden erstgenannten Amine können sich durch Decarboxylierung der Aminosäuren Prolin und Hydroxyprolin bilden. Die Entstehung von Piperidin ist bislang nicht geklärt. Auch im Tierexperiment bekannte, Krebs auslösende Stoffe, wie aromatische Amine, werden erfindungsgemäß durch Klinoptilolith gebunden und so aus dem Speisebrei des Darms entfernt. Hierzu zählen die aromatischen Amine Benzidin und Anilin. Aber auch das 1, 2-Dimethylhydrazin, welches zur tierexperimentellen Auslösung von Colonkrebs benutzt werden kann, wird in seiner Konzentration überraschenderweise durch Bindung an das Klinoptilolith reduziert, was allerdings nicht Gegenstand der Erfindung ist. Damit entfernt Klinoptilolith aus dem Speisebrei sowohl kanzerogene als auch präkanzerogene Stoffe.

Die Reduktion der Konzentration des primären Amins 2-(4'-Imidazolyl)-ethylamin durch Kontakt mit Klinoptilolith im Speisenbrei des Darms sowie auf der Haut und innerhalb von Wunden ist von besonderer Bedeutung, da dieser Stoff im Organismus ein wichtiges Gewebshormon darstellt. Nach bakteriellen Infektionen wird von einer Reihe von Keimen die Decarboxylierung der Aminosäure Histidin genutzt, um innerhalb einer Wunde große Mengen an 2-(4'-Imidazolyl)-ethylamin zu erzeugen. Diese Mengen überfordern die üblichen oxidativen Abbaureaktionen von 2-(4'-Imidazolyl)-ethylamin stark. Es kommt zur intensiven Nesselsucht innerhalb der Wunde. Das vom Körper abgegebene Nesselsekret ist für die Entwicklung bakterieller Erreger ein ausgezeichneter Nährboden, so dass die Infektion innerhalb der Wunde sich deutlich verstärkt. Besonders bei Infektionen mit Methicillin-resistenten *Staphylococcus aureus-*Erregern, die im Folgenden mit der die Abkürzung MRSA bezeichnet werden, ist dieser Sachverhalt bedeutsam, da im Falle von MRSA-Infektionen gegenüber üblichen Antibiotika Resistenzen auftreten, die eine Behandlung sehr erschweren oder gar völlig unmöglich machen können. Indem die gereinigte Wunde mehrfach mit Klinoptilolith oder mit einem Gemisch aus Klinoptilolith und Wasser versetzt und anschließend steril verbunden wird, kann der Mechanismus, der die 2-(4'-Imidazolyl)-ethylamin-Freisetzung, die Bildung von Sesselsekret und die Verstärkung der Infektion umfasst, überraschenderweise durchbrochen werden. Die MRSA-Keime und andere 2-(4'-Imidazolyl)-ethylamin-freisetzende Keime haben somit gegenüber den genetisch "normalen" *Staphylococcus aureus-*Bakterien und anderen nicht 2-(4'-Imidazolyl)-ethylamin-bildenden Keimen keinen Vorteil mehr, den sie bei der Infektion und ihrer Vermehrung innerhalb der Wunde für sich nutzen könnten. Ein Organismus ohne Immundefizite gewinnt relativ schnell die Kontrolle über die Wundheilung. Es kann die Ausheilung beginnen. Eingeleitete Anwendungen an Tieren waren erfolgreich.

Die Reduktion der Konzentration von Cystamin, welches in Form von Bis-(2-aminoethyl)-disulfid-dihydrochlorid vorliegen kann, gelingt ebenfalls mit Klinoptilolith, was allerdings nicht Gegenstand der Erfindung ist. Klinoptilolithaltiges Material mit einem entsprechenden hygienischen Standard ist auch aufgrund der Reduktion der Konzentration von Cystamin, welches, wie bereits erwähnt, einer Blutgerinnung entgegenwirkt, in der Lage, kleinere Blutungen und Schürfwunden sehr rasch zu stoppen. Das Klinoptilolith verbindet sich mit dem sich bildenden Schorf und fällt nach Heilung mit diesem gemeinsam schließlich ab. Die sichtbare Heilungsgeschwindigkeit steigt deutlich an.

Weitere Einzelheiten, Merkmale und Vorteile der Erfindung ergeben sich aus der Zeichnung und der nachfolgenden Beschreibung von Ausführungsbeispielen:
Es zeigt:

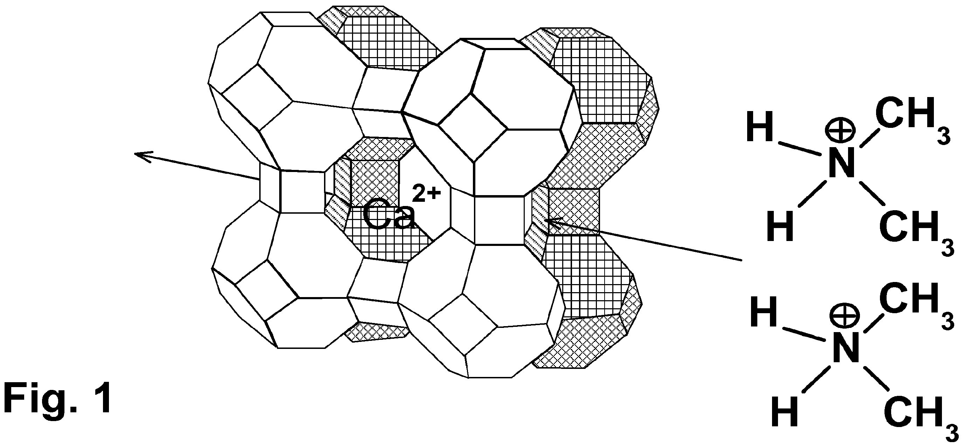

- **Fig. 1:**: eine schematische Darstellung des lonenaustauschs an Klinoptilolith.

### Beispiel 1 (nicht zur Erfindung gehörend)

62,9 mg 2-(4'-Hydroxyphenyl)-ethylamin-hydrochlorid werden zu 50,0 ml in destilliertem Wasser gelöst. 0,500 ml dieser Lösung werden mit destilliertem Wasser so verdünnt, dass die Konzentration an 2-(4'-Hydroxyphenyl)-ethylammonium schließlich 40,0 mg/l beträgt. Diese Lösung versetzt man mit 500 mg Klinoptilolith und rührt bei 20 °C 3,5 Stunden.

Nach Zentrifugation der Reaktionslösung und Klarfiltration des Überstandes wird die Konzentration an 2-(4'-Hydroxyphenyl)-ethylammonium bestimmt. Ergebnis: Die Konzentration an 2-(4'-Hydroxyphenyl)-ethylammonium wurde um 35,2 Prozent erniedrigt.

### Beispiel 2 (nicht zur Erfindung gehörend)

62,9 mg 2-(4'-Hydroxyphenyl)-ethylamin-hydrochlorid werden zu 50,0 ml in destilliertem Wasser gelöst. 0,500 ml dieser Lösung werden mit destilliertem Wasser so verdünnt, dass die Konzentration an 2-(4'-Hydroxyphenyl)-ethylammonium schließlich 40,0 mg/l beträgt. Diese Lösung versetzt man mit 50 mg Klinoptilolith und rührt bei 20 °C 7 Stunden.

Nach Zentrifugation der Reaktionslösung und Klarfiltration des Überstandes wird die Konzentration an 2-(4'-Hydroxyphenyl)-ethylammonium bestimmt. Daraus wird die Erniedrigung der Konzentration an 2-(4'-Hydroxyphenyl)-ethylammonium ermittelt.

Ergebnis: Die Konzentration an 2-(4'-Hydroxyphenyl)-ethylammonium wurde um 13,2 erniedrigt.

### Beispiel 3 (nicht zur Erfindung gehörend)

71,0 mg 2-(4'-Hydroxyphenyl)-ethylamin-hydrochlorid werden zu 50,0 ml in destilliertem Wasser gelöst. 0,500 ml dieser Lösung werden mit destilliertem Wasser so verdünnt, dass die Konzentration an 2-(4'-Hydroxyphenyl)-ethylammonium schließlich 40,0 mg/l beträgt. Diese Lösung versetzt man mit 100 mg Klinoptilolith-Material mit einem Gehalt von 44 Prozent und rührt bei 37 °C 35 Stunden.

Nach Zentrifugation der Reaktionslösung und Klarfilterung des Überstandes wird die Erniedrigung der Konzentration an 2-(4'-Hydroxyphenyl)-ethylammonium bestimmt.

Ergebnis: Die Konzentration an 2-(4'-Hydroxyphenyl)-ethylammonium wurde um 11,8 Prozent erniedrigt.

### Beispiel 4 (nicht zur Erfindung gehörend)

45,3 mg 2-(4'-Hydroxyphenyl)-ethylamin-hydrochlorid werden zu 50,0 ml in destilliertem Wasser gelöst. 0,500 ml dieser Lösung werden mit destilliertem Wasser so verdünnt, dass die Konzentration an 2-(4'-Hydroxyphenyl)-ethylammonium schließlich 40,0 mg/l beträgt. Diese Lösung versetzt man mit 500 mg Klinoptilolith, welches zuvor in der Labormühle sehr fein gemahlen wurde, und rührt bei 37 °C 17 Stunden.

Nach Zentrifugation der Reaktionslösung und Klarfiltration des Überstandes wird die Erniedrigung der Konzentration an 2-(4'-Hydroxyphenyl)-ethylammonium bestimmt.

Ergebnis: Die Konzentration an 2-(4'-Hydroxyphenyl)-ethylammonium wurde um 38,0 Prozent erniedrigt.

### Beispiel 5 (nicht zur Erfindung gehörend)

62,9 mg 2-(4'-Hydroxyphenyl)-ethylamin-hydrochlorid werden zu 50,0 ml in destilliertem Wasser gelöst. 0,500 ml dieser Lösung werden mit destilliertem Wasser so verdünnt, dass die Konzentration an 2-(4'-Hydroxyphenyl)-ethylammonium schließlich 20,0 mg/l beträgt. Diese Lösung versetzt man mit 500 mg Klinoptilolith-Material mit einem Klinoptilolith-Gehalt von 55 Prozent und rührt bei 20 °C 7 Stunden.

Nach Zentrifugation der Reaktionslösung und Klarfiltration des Überstandes wird die Konzentration an 2-(4'-Hydroxyphenyl)-ethylammonium bestimmt. Ergebnis: Die Konzentration an 2-(4'-Hydroxyphenyl)-ethylammonium wurde um 52,7 Prozent erniedrigt.

### Beispiel 6 (nicht zur Erfindung gehörend)

55,7 mg 2-(4'-Hydroxyphenyl)-ethylamin-hydrochlorid werden zu 50,0 ml in destilliertem Wasser gelöst. 0,500 ml dieser Lösung werden mit destilliertem Wasser so verdünnt, dass die Konzentration an 2-(4'-Hydroxyphenyl)-ethylammonium schließlich 20,0 mg/l beträgt. Diese Lösung versetzt man mit 500 mg Klinoptilolith-Material, Klinoptilolith-Gehalt 55 Prozent, schüttelt die Lösung durch und lässt diese über Nacht stehen.

Nach Zentrifugation der Reaktionslösung und Klarfiltration des Überstandes wird die Konzentration an 2-(4'-Hydroxyphenyl)-ethylammonium bestimmt. Ergebnis: Die Konzentration an 2-(4'-Hydroxyphenyl)-ethylammonium wurde um 29,1 Prozent erniedrigt.

### Beispiel 7 (nicht zur Erfindung gehörend)

18,2 mg 1,2-Dimethylhydrazin-dihydrochlorid werden zu 50,0 ml in destilliertem Wasser gelöst.

0,500 ml dieser Lösung werden mit destilliertem Wasser so verdünnt, dass die Konzentration an 1,2-Dimethylhydrazinium-Dikation schließlich 40,0 mg/l beträgt.

Diese Lösung versetzt man mit 500 mg Klinoptilolith-Material mit einem Klinoptilolith-Gehalt von 80 Prozent und rührt bei 20 °C 3 Stunden.

Nach Zentrifugation der Reaktionslösung und Klarfiltration des Überstandes wird die Konzentration des 1,2-Dimethylhydrazinium-Dikations bestimmt. Ergebnis: Die Konzentration an 1,2-Dimethylhydrazinium-Dikation wurde um 87,9 Prozent erniedrigt.

### Beispiel 8 (nicht zur Erfindung gehörend)

18,2 mg 1,2-Dimethylhydrazin-dihydrochlorid werden zu 50,0 ml in destilliertem Wasser gelöst.

0,500 ml dieser Lösung werden mit destilliertem Wasser so verdünnt, dass die Konzentration an 1,2-Dimethylhydrazinium-Dikation schließlich 40,0 mg/l beträgt.

Diese Lösung versetzt man mit 50 mg Klinoptilolith-Material mit einem Klinoptilolith-Gehalt von 89 Prozent und rührt bei 20 °C 7 Stunden.

Nach Zentrifugation der Reaktionslösung und Klarfiltration des Überstandes wird die Konzentration des 1,2-Dimethylhydrazinium-Dikations bestimmt. Ergebnis: Die Konzentration an 1,2-Dimethylhydrazinium-Dikation wurde um 28,5 Prozent erniedrigt.

### Beispiel 9 (nicht zur Erfindung gehörend)

18,2 mg 1,2-Dimethylhydrazin-dihydrochlorid werden zu 50,0 ml in destilliertem Wasser gelöst.

0,500 ml dieser Lösung werden mit destilliertem Wasser so verdünnt, dass die Konzentration an 1,2-Dimethylhydrazinium-Dikation schließlich 10,0 mg/l beträgt.

Diese Lösung versetzt man mit 50 mg technischem Klinoptilolith und rührt bei 37 °C 27 Stunden.

Nach Zentrifugation der Reaktionslösung und Klarfiltration des Überstandes wird die Konzentration des 1,2-Dimethylhydrazinium-Dikations bestimmt. Ergebnis: Die Konzentration an 1,2-Dimethylhydrazinium-Dikation wurde um 48,5 Prozent erniedrigt.

### Beispiel 10 (nicht zur Erfindung gehörend)

18,2 mg 1,2-Dimethylhydrazin-dihydrochlorid werden zu 50,0 ml in destilliertem Wasser gelöst.

0,500 ml dieser Lösung werden mit destilliertem Wasser so verdünnt, dass die Konzentration an 1,2-Dimethylhydrazinium-Dikation schließlich 10,0 mg/l beträgt.

Diese Lösung versetzt man mit 500 mg Klinoptilolith-Material und rührt bei 20 °C 6 Stunden.

Nach Zentrifugation der Reaktionslösung und Klarfiltration des Überstandes wird die Konzentration des 1,2-Dimethylhydrazinium-Dikations bestimmt. Ergebnis: Die Konzentration an 1,2-Dimethylhydrazinium-Dikation wurde um >98 Prozent erniedrigt.

### Beispiel 11 (nicht zur Erfindung gehörend)

19,0 mg Bis-(2-aminoethyl)-disulfid-dihydrochlorid werden zu 15,0 ml in destilliertem Wasser gelöst.

0,500 ml dieser Lösung werden mit destilliertem Wasser so verdünnt, dass die Konzentration an Bis-(2-ammonium-ethyl)-disulfid-Dikation schließlich 40,0 mg/l beträgt.

Diese Lösung versetzt man mit 500 mg Klinoptilolith-Material und rührt bei 20 °C 90 Minuten.

Nach Zentrifugation der Reaktionslösung und Klarfiltration des Überstandes wird die Konzentration an Bis-(2-ammonium-ethyl)-disulfid-Dikation bestimmt. Ergebnis: Die Konzentration an Bis-(2-ammonium-ethyl)-disulfid-Dikation wurde um >99 Prozent erniedrigt.

### Beispiel 12 (nicht zur Erfindung gehörend)

19,0 mg Bis-(2-aminoethyl)-disulfid-dihydrochlorid werden zu 15,0 ml in destilliertem Wasser gelöst.

0,500 ml dieser Lösung werden mit destilliertem Wasser so verdünnt, dass die Konzentration an Bis-(2-ammonium-ethyl)-disulfid-Dikation schließlich 40,0 mg/l beträgt.

Diese Lösung versetzt man mit 50 mg Klinoptilolith-Material und rührt bei 20 °C 90 Minuten.

Nach Zentrifugation der Reaktionslösung und Klarfiltration des Überstandes wird die Konzentration an Bis-(2-ammonium-ethyl)-disulfid-Dikation bestimmt. Ergebnis: Die Konzentration an Bis-(2-ammonium-ethyl)-disulfid-Dikation wurde um 80,6 Prozent erniedrigt.

### Beispiel 13 (nicht zur Erfindung gehörend)

19,0 mg Bis-(2-aminoethyl)-disulfid-dihydrochlorid werden zu 15,0 ml in destilliertem Wasser gelöst.

0,500 ml dieser Lösung werden mit destilliertem Wasser so verdünnt, dass die Konzentration an Bis-(2-ammonium-ethyl)-disulfid-Dikation schließlich 40,0 mg/l beträgt.

Diese Lösung versetzt man mit 50 mg Klinoptilolith-Material mit einem Klinoptilolith-Gehalt von 55 Prozent und rührt bei 37 °C 17 Minuten.

Nach Zentrifugation der Reaktionslösung und Klarfiltration des Überstandes wird die Konzentration an Bis-(2-ammonium-ethyl)-disulfid-Dikation bestimmt. Ergebnis: Die Konzentration des Bis-(2-ammonium-ethyl)-disulfid-Dikations wurde um 92,5 Prozent erniedrigt.

### Beispiel 14 (nicht zur Erfindung gehörend)

19,0 mg Bis-(2-aminoethyl)-disulfid-dihydrochlorid werden zu 15,0 ml in destilliertem Wasser gelöst.

0,500 ml dieser Lösung werden mit destilliertem Wasser so verdünnt, dass die Konzentration an Bis-(2-ammonium-ethyl)-disulfid-Dikation schließlich 10,0 mg/l beträgt.

In diese Lösung streut man über einen Pulvertrichter 50 mg Klinoptilolith, verschließt den Pulvertrichter, schüttelt alles gut durch und lässt diesen bei 20 °C über Nacht stehen.

Nach Zentrifugation der Reaktionslösung und Klarfiltration des Überstandes wird die Konzentration an Cystamin-Dikation bestimmt.

Ergebnis: Die Konzentration des Cystamin-Dikations wurde um 94,0 Prozent erniedrigt.

### Beispiel 15 (nicht zur Erfindung gehörend)

19,0 mg Bis-(2-amino-ethyl)-disulfid-dihydrochlorid werden zu 15,0 ml in destilliertem Wasser gelöst.

0,500 ml dieser Lösung werden mit destilliertem Wasser so verdünnt, dass die Konzentration an Bis-(2-ammonium-ethyl)-disulfid-Dikation schließlich 20,0 mg/l beträgt.

Diese Lösung versetzt man mit 500 mg Klinoptilolith-Material und rührt bei 20 °C 30 Minuten.

Nach Zentrifugation der Reaktionslösung und Klarfiltration des Überstandes wird die Konzentration an Bis-(2-ammonium-ethyl)-disulfid-Dikation bestimmt. Ergebnis: Die Konzentration des Bis-(2-ammonium-ethyl)-disulfid-Dikations wurde um >99 Prozent erniedrigt.

### Beispiel 16 (nicht zur Erfindung gehörend)

15,6 mg Dimethylamin-hydrochlorid werden zu 10,0 ml in destilliertem Wasser gelöst. Beispielhaft ist in **Fig. 1** schematisch der Austausch von Ca²⁺ eines Zeoliths mit einem Dimethylammoniumionen dargestellt.

0,500 ml dieser Lösung werden mit destilliertem Wasser so verdünnt, dass die Konzentration an Dimethylammonium-Kation schließlich 40,0 mg/l beträgt. Diese Lösung versetzt man mit 500 mg Klinoptilolith-Material und rührt bei 20 °C 30 Minuten.

Nach Zentrifugation der Reaktionslösung und Klarfiltration des Überstandes wird die Konzentration an Dimethylammonium bestimmt.

Ergebnis: Die Konzentration von Dimethylammonium wurde um >96,2 Prozent erniedrigt.

### Beispiel 17 (nicht zur Erfindung gehörend)

15,6 mg Dimethylamin-hydrochlorid werden zu 10,0 ml in destilliertem Wasser gelöst.

0,500 ml dieser Lösung werden mit destilliertem Wasser so verdünnt, dass die Konzentration an Dimethylammonium-Kation schließlich 10,0 mg/l beträgt. Diese Lösung versetzt man mit 500 mg Klinoptilolith-Material und rührt bei 20 °C 90 Minuten.

Nach Zentrifugation der Reaktionslösung und Klarfiltration des Überstandes wird die Konzentration an Dimethylammonium bestimmt.

Ergebnis: Die Konzentration von Dimethylammonium wurde um >99 Prozent erniedrigt.

### Beispiel 18 (nicht zur Erfindung gehörend)

15,6 mg Dimethylamin-hydrochlorid werden zu 10,0 ml in destilliertem Wasser gelöst.

0,500 ml dieser Lösung werden mit destilliertem Wasser so verdünnt, dass die Konzentration an Dimethylammonium-Kation schließlich 20,0 mg/l beträgt. Diese Lösung versetzt man mit 500 mg Klinoptilolith-Material und rührt bei 37 °C 19 Stunden.

Nach Zentrifugation der Reaktionslösung und Klarfiltration des Überstandes wird die Konzentration an Dimethylammonium bestimmt.

Ergebnis: Die Konzentration an Dimethylammonium wurde um >99 Prozent erniedrigt.

### Beispiel 19

13,5 mg 2-(4'-Imidazolyl)-ethylamin-dihydrochlorid werden in destilliertem Wasser zu 10,0 ml gelöst. 0,500 ml dieser Lösung werden mit destilliertem Wasser so verdünnt, dass die Konzentration an 2-(4'-Imidazolyl)-ethylammonium-Dikation schließlich 10,0 mg/l beträgt.

Diese Lösung versetzt man mit 500 mg grob zerkleinertem Klinoptilolith-Material mit einem Gehalt von 42 Prozent und rührt bei 20 °C 1 Stunde.

Nach Zentrifugation der Reaktionslösung und Klarfiltration des Überstandes wird die Konzentration an 2-(4'-Imidazolyl)-ethylammonium-Dikation bestimmt. Ergebnis: Die Erniedrigung der Konzentration des 2-(4'-Imidazolyl)-ethylammonium-Dikations beträgt 92 Prozent.

### Beispiel 20 (nicht zur Erfindung gehörend)

13,3 mg Tryptamin-hydrochlorid werden in destilliertem Wasser zu 25,0 ml gelöst. 0,500 ml dieser Lösung werden mit destilliertem Wasser so verdünnt, dass die Konzentration an Tryptamin-Kation 40,0 mg/l beträgt.

Diese Lösung versetzt man mit 500 mg fein pulverisiertem Klinoptilolith und rührt bei 19 °C 1,5 Stunden.

Nach Zentrifugation der Reaktionslösung und Klarfiltration des Überstandes wird die Konzentration an Tryptamin-Kation bestimmt.

Ergebnis: Die Konzentration an Tryptamin-Kation wurde um 79,5 Prozent erniedrigt.

### Beispiel 21

13,5 mg 2-(4'Imidazolyl)-ethylamin-dihydrochlorid werden in destilliertem Wasser zu 10,0 ml gelöst. 0,500 ml dieser Lösung werden mit destilliertem Wasser so verdünnt, dass die Konzentration an 2-(4'-Imidazolyl)-ethylammonium-Dikation schließlich 40,0 mg/l beträgt.

Diese Lösung versetzt man mit 500 mg Klinoptilolith, mit einem Gehalt von 82 Prozent, und rührt bei 21 °C 19 Stunden.

Nach Zentrifugation der Reaktionslösung und Klarfiltration des Überstandes wird die Konzentration an 2-(4'-Imidazolyl)-ethylammonium-Dikation bestimmt. Ergebnis: Die Konzentration an 2-(4'-Imidazolyl)-ethylammonium-Dikation wurde um 84,6 Prozent erniedrigt.

### Beispiel 22

13,5 mg 2-(4'-Imidazolyl)-ethylamin-dihydrochlorid werden in destilliertem Wasser zu 10,0 ml gelöst. 0,500 ml dieser Lösung werden mit destilliertem Wasser so verdünnt, dass die Konzentration an 2-(4'-Imidazolyl)-ethylammonium-Dikation schließlich 40,0 mg/l beträgt.

Diese Lösung versetzt man mit 500 mg Klinoptilolith und rührt bei 20 °C 1,5 Stunden.

Nach Zentrifugation der Reaktionslösung und Klarfiltration des Überstandes wird die Konzentration an 2-(4'-Imidazolyl)-ethylammonium-Dikation bestimmt. Ergebnis: Die Konzentration an 2-(4'-Imidazolyl)-ethylammonium-Dikation wurde um 96,7 Prozent erniedrigt.

### Beispiel 23 (nicht zur Erfindung gehörend)

13,3 mg Tryptamin-hydrochlorid werden in destilliertem Wasser zu 25,0 ml gelöst. 0,500 ml dieser Lösung werden mit destilliertem Wasser so verdünnt, dass die Konzentration an Tryptamin-Kation 40,0 mg/l beträgt.

Diese Lösung versetzt man mit 50 mg fein pulverisiertem Klinoptilolith und rührt bei 19 °C 1,5 Stunden.

Nach Zentrifugation der Reaktionslösung und Klarfiltration des Überstandes wird die Konzentration an Tryptamin-Kation bestimmt.

Ergebnis: Die Konzentration an Tryptamin-Kation wurde um 55,8 Prozent erniedrigt.

### Beispiel 24

13,5 mg 2-(4'-Imidazolyl)-ethylamin-dihydrochlorid werden in destilliertem Wasser zu 10,0 ml gelöst. 0,500 ml dieser Lösung werden mit destilliertem Wasser so verdünnt, dass die Konzentration an 2-(4'-Imidazolyl)-ethylammonium-Dikation schließlich 20,0 mg/l beträgt.

Diese Lösung versetzt man mit 500 mg Klinoptilolith und rührt bei 20 °C 3 Stunden.

Nach Zentrifugation der Reaktionslösung und Klarfiltration des Überstandes wird die Konzentration an 2-(4'-Imidazolyl)-ethylammonium-Dikation bestimmt. Ergebnis: Die Konzentration an 2-(4'-Imidazolyl)-ethylammonium-Dikation wurde um 97,5 Prozent erniedrigt.

### Beispiel 25 (nicht zur Erfindung gehörend)

18,5 mg Tryptamin-hydrochlorid werden in destilliertem Wasser zu 25,0 ml gelöst. 0,500 ml dieser Lösung werden mit destilliertem Wasser so verdünnt, dass die Konzentration an Tryptamin-Kation 20,0 mg/l beträgt.

Diese Lösung versetzt man mit 50 mg fein pulverisiertem Klinoptilolith und rührt bei 19 °C 1,5 Stunden.

Nach Zentrifugation der Reaktionslösung und Klarfiltration des Überstandes wird die Konzentration an Tryptamin-Kation bestimmt.

Ergebnis: Die Konzentration an Tryptamin-Kation wurde um 72,5 Prozent erniedrigt.

### Beispiel 26 (nicht zur Erfindung gehörend)

20,5 mg Tryptamin-hydrochlorid werden in destilliertem Wasser zu 20,0 ml gelöst. 0,500 ml dieser Lösung werden mit destilliertem Wasser so verdünnt, dass die Konzentration an Tryptamin-Kation 10,0 mg/l beträgt.

Diese Lösung versetzt man mit 50 mg Klinoptilolith und rührt bei 37 °C 6 Stunden.

Nach Zentrifugation der Reaktionslösung und Klarfiltration des Überstandes wird die Konzentration an Tryptamin-Kation bestimmt.

Ergebnis: Die Konzentration an Tryptamin-Kation wurde um 89,9 Prozent erniedrigt.

### Beispiel 27 (nicht zur Erfindung gehörend)

89,5 mg Dimethylamin-hydrochlorid werden zu 50,0 ml in destilliertem Wasser gelöst.

0,500 ml dieser Lösung werden mit destilliertem Wasser so verdünnt, dass die Konzentration an Dimethylammonium-Kation schließlich 40,0 mg/l beträgt. Diese Lösung versetzt man mit 50 mg Klinoptilolith und rührt bei 20 °C 90 Minuten.

Nach Zentrifugation der Reaktionslösung und Klarfiltration des Überstandes wird die Konzentration an Dimethylammonium bestimmt.

Ergebnis: Die Konzentration von Dimethylammonium wurde um 32,9 Prozent erniedrigt.

### Beispiel 28 (nicht zur Erfindung gehörend)

54,4 mg 2-Phenylethylamin-hydrochlorid werden zu 50,0 ml in destilliertem Wasser gelöst.

0,500 ml dieser Lösung werden mit destilliertem Wasser so verdünnt, dass die Konzentration an Dimethylammonium-Kation schließlich 40,0 mg/l beträgt. Diese Lösung versetzt man mit 50 mg Klinoptilolith und rührt bei 20 °C 90 Minuten.

Nach Zentrifugation der Reaktionslösung und Klarfiltration des Überstandes wird die Konzentration an 2-Phenylethyl-ammonium bestimmt.

Ergebnis: Die Konzentration von 2-Phenylethyl-ammonium wurde um 13,5 Prozent erniedrigt.

### Beispiel 29 (nicht zur Erfindung gehörend)

54,4 mg 2-Phenylethylamin-hydrochlorid werden zu 50,0 ml in destilliertem Wasser gelöst.

0,500 ml dieser Lösung werden mit destilliertem Wasser so verdünnt, dass die Konzentration an 2-Phenylethyl-ammonium-Kation schließlich 40,0 mg/l beträgt.

Diese Lösung versetzt man mit 500 mg Klinoptilolith und rührt bei 20 °C 90 Minuten.

Nach Zentrifugation der Reaktionslösung und Klarfiltration des Überstandes wird die Konzentration an 2-Phenylethyl-ammonium bestimmt.

Ergebnis: Die Konzentration von 2-Phenylethyl-ammonium wurde um 75,2 Prozent erniedrigt.

### Beispiel 30

13,5 mg 2-(4'-Imidazolyl)-ethylamin-dihydrochlorid werden in destilliertem Wasser zu 10,0 ml gelöst. 0,500 ml dieser Lösung werden mit destilliertem Wasser so verdünnt, dass die Konzentration an 2-(4'-Imidazolyl)-ethylammonium-dichlorid schließlich 30,0 mg/l beträgt.

Diese Lösung versetzt man mit 100 mg Klinoptilolith und rührt bei 20 °C 30 Minuten.

Nach Zentrifugation der Reaktionslösung und Klarfiltration des Überstandes wird die Konzentration an 2-(4'-Imidazolyl)-ethylammonium-Dikation bestimmt. Ergebnis: Die Konzentration an 2-(4'-Imidazolyl)-ethylammonium-Dikation wurde um >99 Prozent erniedrigt.

### Beispiel 31

52,8 mg 2-(4'-Imidazolyl)-ethylamin-dihydrochlorid werden in destilliertem Wasser zu 50,0 ml gelöst. 0,500 ml dieser Lösung werden mit destilliertem Wasser so verdünnt, dass die Konzentration an 2-(4'-Imidazolyl)-ethylammonium-dichlorid schließlich 30,0 mg/l beträgt.

Diese Lösung versetzt man mit 75 mg Klinoptilolith und rührt bei 20 °C 40 Minuten.

Nach Zentrifugation der Reaktionslösung und Klarfiltration des Überstandes wird die Konzentration an 2-(4'-Imidazolyl)-ethylammonium-Dikation bestimmt. Ergebnis: Die Konzentration an 2-(4'-Imidazolyl)-ethylammonium-Dikation wurde um 91,7 Prozent erniedrigt.

### Beispiel 32

13,5 mg 2-(4'-Imidazolyl)-ethylamin-dihydrochlorid werden in destilliertem Wasser zu 10,0 ml gelöst. 0,500 ml dieser Lösung werden mit destilliertem Wasser so verdünnt, dass die Konzentration an 2-(4'-Imidazolyl)-ethylammonium-dichlorid schließlich 30,0 mg/l beträgt.

Eine derartige Lösung versetzt man mit 100 mg Klinoptilolith und schüttelt diese bei 20 °C 30 Minuten.

Nach Zentrifugation der Reaktionslösung und Klarfiltration des Überstandes wird die Konzentration an 2-(4'-Imidazolyl)-ethylammonium-Dikation bestimmt. Ergebnis: Die Konzentration an 2-(4'-Imidazolyl)-ethylammonium-Dikation wurde um >99 Prozent erniedrigt.

### Beispiel 33 (nicht zur Erfindung gehörend)

In einem 50 ml-Maßkolben werden 114,5 mg Pyrrolidin eingewogen. Man gibt nach Verdünnung mit destilliertem Wasser 116 mg Essigsäure zu und füllt bis zur Marke auf.

Man verdünnt 0,800 ml der Lösung so, dass eine Konzentration von Pyrrolidinium von 40 mg/l entsteht.

12,5 ml der Lösung werden mit 0,5 g Klinoptilolith mit der Bezeichnung MANC versetzt und die Mischung wird 90 Minuten bei Zimmertemperatur geschüttelt. Nach Zentrifugation der Reaktionslösung und Klarfiltration des Überstandes wird die Konzentration an Pyrrolidinium bestimmt.

Ergebnis: Die Konzentration an Pyrrolidinium wurde um 76,9 Prozent erniedrigt.

### Beispiel 34 (nicht zur Erfindung gehörend)

In einem 50 ml-Maßkolben werden 9,5 mg Anilin eingewogen. Man gibt nach Verdünnung mit destilliertem Wasser die erforderliche äquivalente Menge an reinster Salzsäure zu und füllt bis zur Marke auf. Man verdünnt 0,500 ml der Lösung so, dass eine Konzentration von Anilinium von 40 mg/l entsteht und versetzt einen aliquoten Teil dieser Lösung mit 0,5 g Klinoptilolith mit der Bezeichnung MANC und rührt die Mischung 120 Minuten bei Zimmertemperatur.

Nach sorgfältiger Abtrennung der Reaktionslösung und Klarfiltration des Überstandes wird die Konzentration an Anilin bestimmt.

Ergebnis: Die Konzentration an Anilinium wurde um 49 Prozent erniedrigt.

### Beispiel 35 (nicht zur Erfindung gehörend)

In einem 50 ml-Maßkolben werden 17,65 mg Benzidin-dihydrochlorid eingewogen. Man gibt nach Verdünnung destilliertes Wasser zu und füllt bis zur Marke auf. Anschließend verdünnt man 0,500 ml der Lösung so, dass eine Konzentration von Benzidinium-Dikation von 40 mg/l entsteht und versetzt die Lösung mit 0,5 g Klinoptilolith und rührt die Mischung 90 Minuten bei Zimmertemperatur.

Nach sorgfältiger Abtrennung der Reaktionslösung und Klarfiltration des Überstandes wird die Konzentration an Benzidin bestimmt.

Ergebnis: Die Konzentration an Benzidin wurde um 98,4 Prozent erniedrigt.

## Patentansprüche

1. Klinoptilolith oder klinoptilolithhaltige Gemische, wobei das Klinoptilolith oder die klinoptilolithhaltigen Gemische durch Zerkleinerung der Teilchen mechanisch modifiziert und aktiviert sind, zur Verwendung als Medizinprodukt zur Aufnahme und Ausleitung von 2-(4'-Imidazolyl)-ethylamin und/oder dessen Salzen, das/die in wässriger Lösung oder wässriger Suspension im Verdauungstrakt oder dem Hautbereich von Mensch und Tier vorkommt/vorkommen.

## Claims

1. Clinoptilolite or mixtures containing clinoptilolite, wherein the clinoptilolite or the mixtures containing clinoptilolite are mechanically modified and activated by granulation of the particles, for use as a medicine product for absorbing and removing 2-(4'-imidazolyle)-ethylamine and/or salts thereof, which is/are present in aqueous solution or aqueous suspension in the digestive tract or the skin region of humans and animals.

## Revendications

1. Clinoptilolite ou mélanges contenant de la clinoptilolite, la clinoptilolite ou les mélanges contenant de la clinoptilolite étant modifiés mécaniquement et activés par le broyage des particules, en vue d'une utilisation comme produit médicinal pour l'absorption et l'évacuation de la 2-(4'-imidazolyl)éthylamine et/ou de ses sels qui est présente/sont présents dans une solution aqueuse ou dans une suspension aqueuse dans l'appareil digestif ou dans la région cutanée des êtres humains ou des animaux.
